# EUROPÄISCHE PATENTSCHRIFT

(11) **EP 1 682 513 B1**
(45) Veröffentlichungstag und Bekanntmachung des Hinweises auf die Patenterteilung: **12.01.2011**
(21) Anmeldenummer: 04790788.6
(22) Anmeldetag: 23.10.2004
(51) Int. Cl.: C07D 217/08, C07C 217/28, C07C 309/32

(54) **VERFAHREN ZUR HERSTELLUNG VON CYCLISCHEN N-SUBSTITUIERTEN ALPHA-IMINOCARBONS UREN**
METHOD FOR PRODUCING CYCLIC, N-SUBSTITUTED ALPHA-IMINOCARBOXYLIC ACIDS
PROCEDE POUR PRODUIRE DES ACIDES ALPHA-IMINOCARBOXYLIQUES CYCLIQUES N-SUBSTITUES

(30) Priorität: 06.11.2003 DE 10351904
(43) Veröffentlichungstag der Anmeldung: 26.07.2006
(73) Patentinhaber: Sanofi-Aventis Deutschland GmbH, 65929 Frankfurt am Main (DE)
(72) Erfinder: SCHUBERT, Gerrit, 65779 Kelkheim (DE)
(86) Internationale Anmeldenummer: PCT/EP2004/011997
(87) Internationale Veröffentlichungsnummer: WO 2005/047263

(56) Entgegenhaltungen:
- WO-A-97/18194

## Beschreibung

Die Erfindung betrifft ein Verfahren zur Herstellung von cyclischen N-substituierten alpha-Iminocarbonsäuren der Formel I. Die Verbindungen der Formel I sind selektive Inhibitoren der Kollagenase (MMP 13) und können daher zur Behandlung degenerativer Gelenkerkrankungen eingesetzt werden.

Es ist bekannt, dass cyclische und heterocyclische N-substituierten alpha-Iminohydroxam- und Carbonsäuren Inhibitoren von Metalloproteinasen sind (EP 0 861 236). Bekannte Verfahren zur Herstellung dieser Verbindungen haben eine hohe Stufenzahl, relativ geringe Ausbeute, verwenden krebserregende Zwischenverbindungen wie Nitro-Biphenyle oder führen zu Racematen oder teilsweise racemisierten Verbindungen, die anschließend wieder in die Enantiomere aufgetrennt werden müssen. Außerdem müssen bei den bekannten Verfahren die Zwischenstufen oft durch Säulenchromatographie gereinigt werden, wodurch die Synthese großer Substanzmengen im Technikum oder Produktionsbetrieb erschwert wird.

Es wurde nun gefunden, dass sich die genannten Nachteile durch eine kurze, effiziente und racemisierungsfreie Synthese vermeiden lassen, die auch auf aufwendige Reinigungsschritte wie Säulenchromatographie verzichtet.

Die Aufgabe wird dadurch gelöst, dass Biphenyl-carbaminsäureester der Formel II sulfoniert und die gebildeten Sulfonsäuren als Alkalimetallsalze ausgefällt werden. Anschließend erfolgt eine Halogenierung in Anwesenheit einer organischen Base. Durch dieses zweistufige Verfahren gelingt die Herstellung der Sulfonsäurehalogenide, die aufgrund der sauer katalysierten Zersetzungsreaktion der Carbaminsäureesterfunktion nicht oder nur schlecht direkt in einer Stufe chlorsulfoniert werden können. Die Herstellung der Verbindungen der Formel I erfolgt dann durch Umsetzung mit einer Verbindung V, die zunächst mit einem Silierungsmittel silyliert wird.

Die Erfindung betrifft daher ein Verfahren zur Gewinnung der Verbindung der Formel I 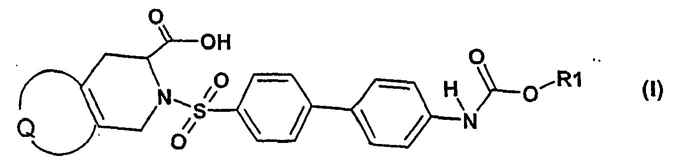 und/oder alle stereoisomeren Formen der Verbindung der Formel I und/oder Gemische diese Formen in jedem Verhältnis, und/oder ein physiologisch verträgliches Salz der Verbindung der Formel I, wobei
R1 für
1. -(C₁-C₁₀)-Alkyl, worin Alkyl gerade oder verzweigt ist,
2. -(C₂-C₁₀)-Alkenyl, worin Alkenyl gerade oder verzweigt ist,
3. -(C₂-C₁₀)-Alkinyl, worin Alkinyl gerade oder verzweigt ist,
4. -(C₁-C₄)-Alkyl-Phenyl,
5. -(C₁-C₄)-Alkyl-(C₃-C₇)-Cycloalkyl,
6. -(C₃-C₇)-Cycloalkyl oder
7. -CH₂CF₃ steht und
und worin 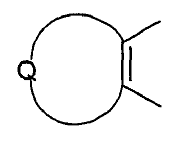 ein Rest aus der Reihe 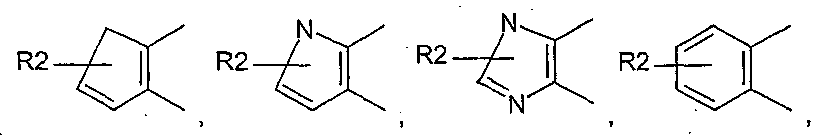 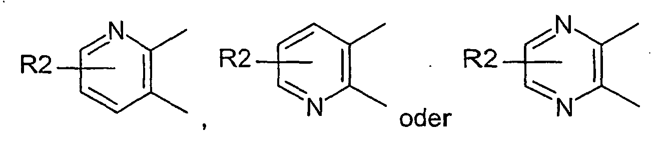 ist und unsubstituiert oder ein-, zwei- oder dreifach substituiert ist durch R2, und
R2 für Wasserstoffatom oder -(C₁-C₄)-Alkyl steht,
das dadurch gekennzeichnet ist, dass man
a) eine Verbindung der Formel II 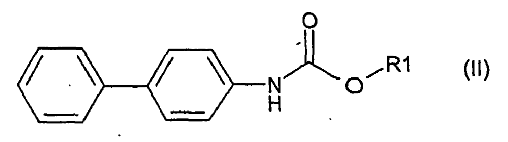 worin R1 die gleiche Bedeutung wie in der Formel I hat,
   mit einem Sulfonierungsreagenz in einem organischen Lösungsmittel umsetzt und mit einer wässrigen Alkalimetallsalz-Lösung in eine Verbindung der Formel III überführt, 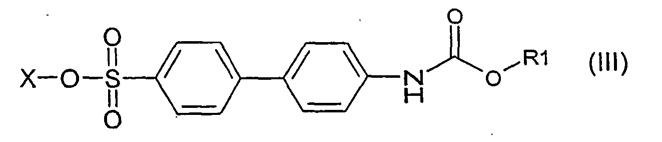 worin X für ein Alkalimetall oder Wasserstoff steht, und
b) die Verbindung der Formel III mit einem Chlorierungsreagenz in einem organischen Lösungsmittel II in Anwesenheit einer organischen Base in eine Verbindung der Formel IV überführt, 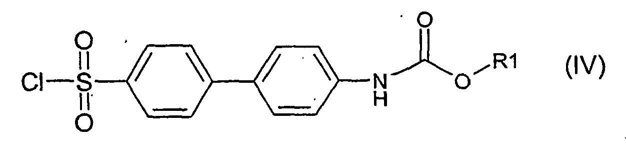 und
c) eine Verbindung der Formel V 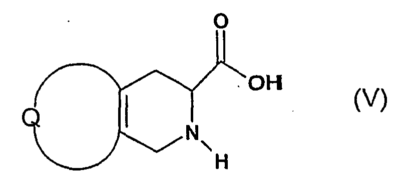 in einem organischen Lösungsmittel III mit einem Silylierungsmittel umsetzt und anschließend mit der Verbindung der Formel IV in eine Verbindung der Formel I überführt.

Unter dem Begriff "(C₁-C₁₀)-Alkyl" werden Kohlenwasserstoffreste verstanden, deren Kohlenstoffkette geradkettig oder verzweigt ist und 1 bis 10 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutyl, Neohexyl, Heptyl, Octanyl, Nonanyl oder Decanyl.

Unter dem Begriff "(C₂-C₁₀)-Alkenyl" werden Kohlenwasserstoffreste verstanden, deren Kohlenstoffketten geradkettig oder verzweigt ist und 1 bis 10 Kohlenstoffatome enthält und je nach Kettenlänge 1, 2 oder 3 Doppelbindungen enthalten. Unter dem Begriff "(C₂-C₁₀)-Alkinyl" werden Kohlenwasserstoffreste verstanden, deren Kohlenstoffketten geradkettig oder verzweigt ist und 1 bis 10 Kohlenstoffatome enthält und je nach Kettenlänge 1, 2 oder 3 Dreifachbindungen enthalten. (C₃-C₇)-CycloalkylReste sind beispielsweise Verbindungen, die sich von 3- bis 7-gliedrige Monocyclen wie Cyclopropyl, Cyclobutyl, Cyclopentyl, Cyclohexyl oder Cycloheptyl herleiten.

Unter dem Begriff "Sulfonierungsreagenz" werden Verbindungen verstanden, wie sie beispielsweise in M.B. Smith and J. March, March's Advanced Organic Chemistry: Reactions, Mechanisms, and Structure, Wiley, New York, 2001, S. 702-704 und der dort zitierten Literatur beschrieben werden, beispielsweise Chlorsulfonsäure, Schwefelsäure, Sulfurylchlorid, rauchende Schwefelsäule oder Schwefeltrioxid oder Gemische davon.

Unter dem Begriff "organisches Lösungsmittel" werden aprotische Lösungsmittel, beispielsweise Chloroform, Dichlormethan, Pentan, Heptan, Hexan, Tetrachlormethan, Benzol Toluol, Xylol, Chlorbenzol, 1,2-Dichlorethan, Trichlorethylen verstanden.

Unter dem Begriff "wässrigen Alkalimetallsalz-Lösung" werden Lösungen von wasserlöslichen Alkalimetallsalzen, beispielsweise Alkalimetallhalogenide wie NaCl, KCl, LiCl, RbCl oder CsCl oder Alkalimetallsulfate wie Na₂SO₄, K₂SO₄, Li₂SO₄, Rb₂SO₄ oder Cs₂SO₄ verstanden. In einem Liter Wasser werden beispielsweise von 10 g bis 300 g NaCl bei 25 ° C gelöst.

Unter dem Begriff "organischen Lösungsmittel II" werden aprotische Lösungsmittel wie beispielsweise Chloroform, Dichlormethan, Pentan, Heptan, Hexan, Tetrachlormethan, Toluol, Benzol, Xylol, Chlorbenzol, 1,2-Dichlorethan, Trichlorethylen verstanden. Unter dem Begriff "organische Base" werden organische Amine wie beispielsweise Chinolin, Morpholin, Piperidin, Pyridin, Triethylamin, Picolin, Lutidin verstanden. Bevorzugt ist Pyridin.

Unter dem Begriff "Chlorierungsreagenz" werden Verbindungen verstanden, die zur Umsetzung von Carbonsäuren, Sulfonsäuren oder deren Salzen zu Carbonsäure- oder Sulfonsäurechloriden verwendet werden können wie beispielsweise PCl₅, POCl₃, SOCl₂, Triphenylphosphin in CCl₄ oder Gemische der Chlorierungsreagenzien. Solche Reagenzien werden beispielsweise in R.C. Larock, Comprehensive Organic Transformations: a Guide to Functional Group Preparations, VCH Publishers, New York, Weinheim, 1999, S. 1929-1930 beschrieben.

Unter dem Begriff "organischen Lösungsmittel III" werden aprotische Lösungsmittel wie beispielsweise Acetonitril, Dichlormethan, Chloroform, Tetrahydrofuran, Toluol, Dimethoxyethan, Dioxan, Diethylenglycoldimethylether verstanden.

Unter dem Begriff "Silylierungsmittel" werden Verbindungen verstanden, die zur Silylierung von Carboxylgruppen geeignet sind, beispielsweise beschrieben in T.W. Greene. P.G.M. Wuts, Protective Groups in Organic Chemistry, Wiley, New York, 1991, Kapitel 5. Beispiele für Silylierungsmittel sind N,O-Bis(trimethylsilyl)-acetamid oder Trimethylsilylchlorid oder Gemische der Silylierungsmittel.

Bei der Herstellung der Verbindung der Formel I geht man so vor, dass zunächst die Verbindung der Formel II in einem organischen Lösungsmittel unter Rühren mit einem Sulfonierungsreagenz umgesetzt wird. Die Reaktionstemperatur beträgt dabei von -40 °C bis +20 °C, bevorzugt von -30 °C bis +20 °C. Die Reaktionszeit liegt im allgemeinen im Bereich von 0,5 bis 6 Stunden, je nach Zusammensetzung des Gemisches und des gewählten Temperaturbereiches. Anschließend wird mit einer wässrigen Alkalimetallsalz-Lösung die erhaltene Verbindung der Formel III ausgefällt. Die Verbindung der Formel III wird anschließend durch Filtration aus dem Reaktionsgemisch abgetrennt.

Es werden vorzugsweise von 0,1 bis 5 Mol der Verbindung der Formel II in 1000 mL organischem Lösungsmittel gelöst. Vorzugsweise verwendet man auf 1 Mol der Verbindung der Formel II von 1 Mol bis 10 Mol, insbesondere von 1 Mol bis 3 Mol, des Sulfonierungsreagenz. Bevorzugte Sulfonierungsreagenzien sind Chlorsulfonsäure, Schwefelsäure, Sulfürylchlorid, rauchende Schwefelsäure oder Schwefeltrioxid. Für die Fällung verwendet man von 0,1 L bis 10 L der wässrigen Alkalimetallsalz-Lösung bezogen auf 1 L des Reaktionsansatzes mit der Verbindung der Formel II. Die Konzentration der Alkalimetallsalz-Lösung beträgt von 10 bis 300 g pro Liter Wasser, wobei die Gesamtmenge an Alkalimetallsalz mindestens 1 Mol auf 1 Mol der organischen Verbindung beträgt.

Die Filtration erfolgt beispielsweise mit einer Büchner-Nutsche und einer Saugflasche unter Anlegen von verminderten Druck oder mit einem Druckfilter.

Die Verbindungen der Formel II sind entweder bekannt oder lassen sich beispielsweise herstellen, indem man Biphenyl-4-isocyanat in einem organischen Lösungsmittel mit dem entsprechenden Alkohol R1-OH umsetzt.

Anschließend werden die Verbindung der Formel III und eine organische Base in einem organischen Lösungsmittel II vorgelegt und unter Rühren mit einem Chlorierungsreagenz umgesetzt. Die Reaktionstemperatur beträgt dabei von 10 °C bis 150 °C, bevorzugt von 30 °C bis 100 °C. Die Reaktionszeit liegt im allgemeinen von 0,5 bis 6 Stunden, je nach Zusammensetzung des Gemisches und des gewählten Temperaturbereiches. Die erhaltene Verbindung der Formel IV wird anschließend durch wässrige Aufarbeitung und Extraktion mit einem Lösungsmittel aus dem Reaktionsgemisch abgetrennt.

Es werden vorzugsweise von 0,2 bis 10 Mol der Verbindung der Formel III in 1000 mL organischem Lösungsmittel II gelöst. Vorzugsweise verwendet man auf 1 Mol der Verbindung der Formel III von 0,1 Mol bis 5 Mol, insbesondere von 0,1 Mol bis 1 Mol, der organischen Base. Vorzugsweise verwendet man auf 1 Mol der Verbindung der Formel III von 1 Mol bis 5 Mol, insbesondere von 1 Mol bis 2 Mol, des Chlorierungsreagenzes. Bevorzugte Chlorierungsreagenzien sind PCl₅, POCl₃, SOCl₂, Triphenylphosphin in CCl₄ oder Gemische, dieser Reagenzien. Die Abtrennung der Verbindung der Formel IV erfolgt durch wässrige Aufarbeitung und Extraktion des Produktes mit einem organischen Lösungsmittel.

Anschließend wird die Verbindung der Formel V in einem organischen Lösungsmittel III vorgelegt und unter Rühren mit einem Silylierungsmittel umgesetzt. Die Reaktionstemperatur beträgt dabei von 10 °C bis 150 °C, bevorzugt von 30 °C bis 100 °C. Die Reaktionszeit liegt im allgemeinen von 0,5 bis 10 Stunden, je nach Zusammensetzung des Gemisches und des gewählten Temperaturbereiches. Danach werden die silylierten Verbindungen der Formel V in demselben Reaktionsgemisch mit den Verbindungen der Formel IV umgesetzt. Dazu werden die Verbindungen IV entweder als Reinsubstanz oder in einem organischen Lösungsmittel III gelöst zugegeben. Die Reaktionstemperatur beträgt dabei von 10 °C bis 150 °C, bevorzugt von 30 °C bis 100 °C. Die Reaktionszeit beträgt im allgemeinen 0,5 bis 10 Stunden, je nach Zusammensetzung des Gemisches und des gewählten Temperaturbereiches. Die erhaltene Verbindung der Formel I wird anschließend durch wässrige Aufarbeitung und Extraktion mit einem organischen Lösungsmittel aus dem Reaktionsgemisch abgetrennt.

Es werden vorzugsweise von 0,1 bis 10 Mol der Verbindung der Formel V in 1000 mL organischem Lösungsmittel III gelöst. Vorzugsweise verwendet man auf 1 Mol der Verbindung der Formel IV von 0,5 Mol bis 2 Mol, insbesondere von 0,9 Mol bis 1,1 Mol, der Verbindung der Formel V. Vorzugsweise verwendet man auf 1 Mol der Verbindung der Formel V von 1 Mol bis 5 Mol, insbesondere von 2 Mol bis 2,5 Mol, des Silylierungsmittels. Bevorzugte Silylierungsmittel sind N,O-Bis-trimethylsilylacetamid oder Trimethylsilylchlorid.

Ein weiterer Aspekt der Erfindung betrifft neue Verbindungen der Formel II, 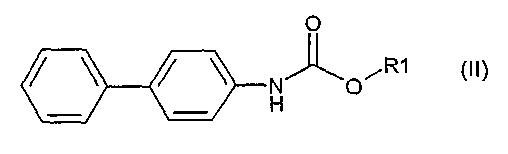 und/oder alle stereoisomeren Formen der Verbindung der Formel II und/oder Gemische diese Formen in jedem Verhältnis, und/oder ein physiologisch verträgliches Salz der Verbindung der Formel II, wobei
R1 für
1. -(C₁-C₁₀)-Alkyl, worin Alkyl gerade oder verzweigt ist,
2. -(C₂-C₁₀)-alkenyl, worin Alkenyl gerade oder verzweigt ist,
3. -(C₂-C₁₀)-Alkinyl, worin Alkinyl gerade oder verzweigt ist,
4. -(C₁-C₄)-Alkyl-Phenyl,
5. -(C₁₋C₄)-Alkyl-(C₃₋C₇)-Cycloalkyl,
6. -(C₃-C₇)-Cycloalkyl oder
7. -CH₂CF₃ steht.

Ein weiterer Aspekt der Erfindung betrifft neue Verbindungen der Formel III, 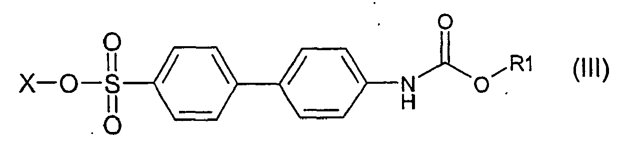 und/oder alle stereoisomeren Formen der Verbindung der Formel III und/oder Gemische diese Formen in jedem Verhältnis, und/oder ein physiologisch verträgliches Satz der Verbindung der Formel III, wobei
R1 für
1. -(C₁-C₁₀)-Alkyl, worin Alkyl gerade oder verzweigt ist,
2. -(C₂-C₁₀)-Alkenyl, worin Alkenyl gerade oder verzweigt ist,
3. -(C₂-C₁₀)-Alkinyl, worin Alkinyl gerade oder verzweigt ist,
4. -(C₁-C₄)-Alkyl-Phenyl,
5. -(C₁-C₄)-Alkyl-(C₃-C₇)-Cycloalkyl,
6. -(C₃-C₇)-Cycloalkyl oder
7. -CH₂CF₃ steht und
X für Li, Na, K, Rb, Cs steht.

Ein weiterer Aspekt der Erfindung betrifft neue Verbindungen der Formel IV, 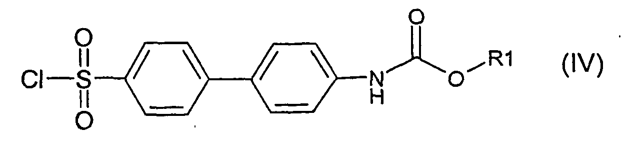 und/oder alle stereoisomeren Formen der Verbindung der Formel IV und/oder Gemische diese Formen in jedem Verhältnis, und/oder ein physiologisch verträgliches Salz der Verbindung der Formel IV, wobei R1 für
1. -(C₁-C₁₀)-Alkyl, worin Alkyl gerade oder verzweigt ist,
2. -(C₂-C₁₀)-Alkenyl, worin Alkenyl gerade oder verzweigt ist,
3. -(C₂-C₁₀)-Alkinyl, worin Alkinyl gerade oder verzweigt ist,
4. -(C₁-C₄)-Alkyl-Phenyl,
5. -(C₁-C₄)-Alkyl-(C₃-C₇)-Cycloalkyl,
6. -(C₃-C₇)-Cycloalkyl oder
7. -CH₂CF₃ steht.

Die Verbindungen der Formeln II, III und IV eignen sich als Zwischenverbindungen zur Synthese der Verbindungen der Formel I, die selektive Inhibitoren der Kollagenase (MMP 13) sind.

Nachfolgend ist die Erfindung an Hand von Beispielen näher erläutert.

Endprodukte werden in der Regel durch ¹H-NMR (400 MHz, in DMSO-D6) bestimmt, angegeben sind jeweils der Hauptpeak oder die beiden Hauptpeaks. Temperaturangaben in Grad Celsius, RT bedeutet Raumtemperatur (22 °C bis 26 °C). Verwendete Abkürzungen sind entweder erläutert oder entsprechen den üblichen Konventionen.

### Beispiel 1

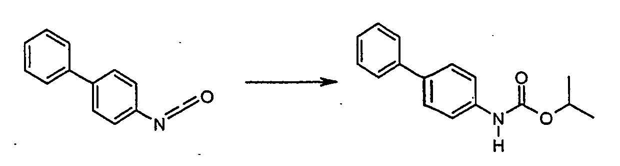

### Herstellung von Biphenyl-4-yl-carbaminsäure-isopropylester

Zu einer Suspension von Biphenyl-4-isocyanat (204 g, Reinheit 96,6 %, 1,0 Mol) in 1,2 Liter (L) Toluol wurde innerhalb von 2 Minuten (Min.) bei 20 °C 230 mL Isopropanol (3 Mol) zugegeben. Die Innentemperatur wurde gegebenenfalls durch leichtes Kühlen mit einem Eisbad unter 30 °C gehalten. Nach 75 Min. wurde das Gemisch auf 50 °C erwärmt und anschließend unter Rühren innerhalb von 2 Stunden (h) auf 20 °C abkühlen gelassen, wobei das Produkt auskristallisierte. Anschließend wurde das Gemisch im Eisbad gekühlt und die abgeschiedenen Kristalle abgesaugt und mit Toluol gewaschen. Man erhielt 188 g kristallines Produkt. Das Filtrat wurde unter verminderten Druck eingeengt und der Rückstand wurde in Toluol (200 mL) zur Kristallisation gebracht. Auf diese Weise wurden weitere 59 g Produkt erhaltene. Gesamtausbeute: 247 g (97 %) Biphenyl-4-yl-carbaminsäure-isopropylester, farblose Kristalle,
Schmelzpunkt (Fp.) 138,5 °C bis 139 °C,
¹H-NMR (400 MHz, CDCl₃): δ = 1.31 (d, J= 6 Hz, 6 H), 5.04 (heptett, J=6 Hz, 1 H), 6.61 (bs, 1 H), 7.31 (t, J= 7.5 H, 1 H), 7.41 (d, J= 8 Hz, 2 H), 7.45 (d, J= 9 Hz, 2 H), 7.55 (t, J= 8 Hz, 2 H) ppm.
Analyse: C₁₆H₁₇NO₂ (255.32): berechnet C 75.27, H 6.71, N 5.49; gefunden C 75.27, H 6.61, N 5.57

### Beispiel 2

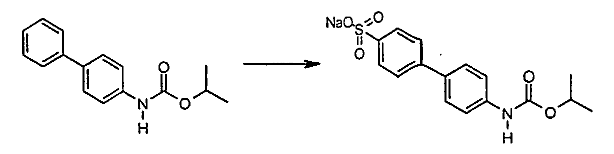

Herstellung von 4'-Isopropoxycarbonylamino-biphenyl-4-sulfonsäure Natriumsalz Eine Suspension von Biphenyl-4-yl-carbaminsäure-isopropylester (256 g, 1 Mol) in Dichlormethan (2000 mL) wurde auf -10 °C gekühlt und unter Rühren wurden innerhalb von 25 Min. Chlorsulfonsäure (350 g, 200 mL, 3 Mol) unter leichter Kühlung mit einem Trockeneis-Methanol-Bad zugetropft. Die Innentemperatur wurde dabei unterhalb von - 4 °C gehalten. Anschließend wurde das Trockeneis-Methanol-Bad durch ein Eisbad ersetzt und das Gemisch 1,5 h lang bei 3 °C bis 14 °C gerührt. Danach wurde bei 2 °C bis 3 °C nochmals Chlorsulfonsäure (175 mL, 2,6 Mol) zugesetzt, erneut 30 Min. nachgerührt und dann aufgearbeitet. Dazu wurde eine Mischung von Eis (5 kg), Methylenchlorid (1000 mL) und konzentrierter NaCl-Lösung (1300 mL) unter starkem Rühren vorgelegt und das Reaktionsgemisch langsam zugegeben. Der ausgefallene Feststoff wurde abgesaugt und unter verminderten Druck bei 45 °C getrocknet. Man erhilt 329 g (92 %) 4'-Isopropoxycarbonylamino-biphenyl-4-sulfonsäure Natriumsalz als weißes, kristallines Pulver, Fp. >260 °C,
¹H-NMR (400 MHz, DMSO-D6): δ = 1.27 (d, J= 6 Hz, 6 H), 4.91 (heptett, J= 6 Hz), 1 H), 7.54-7.66 (sh, 8 H), 9.66 (bs, 1 H) ppm.
Analyse: C₁₆H₁₆NNaO₅S (357.36): berechnet C 53.78, H 4.51, N 3.92, Na 6.43; gefunden C 54.13, H 4.39, N 4.17, Na 5.9.

### Beispiel 3

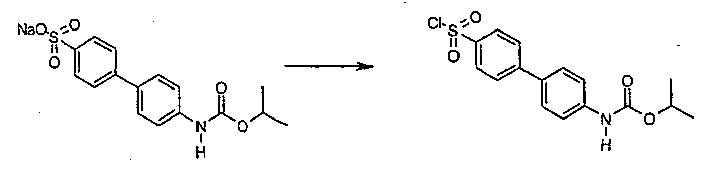

### Herstellung von (4'-Chlorsulfonyl-biphenyl-4-yl)-carbaminsäure isopropylester

Ein Gemisch von 4'-Isopropoxycarbonylamino-biphenyl-4-sulfonsäure Natriumsalz ( 283 g, 0,79 Mol), Toluol (700 mL) und Pyridin (35 mL, 0.43 Mol) wurde bei RT stark gerührt und POCl₃ (215 mL) wurde langsam zugetropft, so dass die Innentemperatur zwischen 30 °C und 45 °C gehalten wurde. Anschließend wurde PCl₅ (100 g) bei 35 °C in etwa 6 Portionen zugesetzt und das Gemisch innerhalb von 30 Min. auf 60 °C erwärmt. Danach wurde bei 70 °C innerhalb von 15 Min. weiteres PCl₅ (100 g) zugegeben und das Gemisch ohne weiteres Erhitzen 1 h lang rühren gelassen. Dabei fiel die Innentemperatur auf etwa 30 °C ab. Man verdünnte mit Dichlormethan (500 mL) und arbeitete dann auf. Dazu wurde ein Gemisch von Eiswasser (5 kg), gesättigter NaCl-Lösung (1000 mL) und Dichlormethan (1000 mL) unter starkem Rühren vorgelegt und das Reaktionsgemisch langsam zugetropft. Die Temperatur wurde dabei durch Zugabe von Eis (2 kg) auf 15 °C bis 25 °C gehalten. Anschließend wurde noch 1 h nachgerührt, wobei man die Temperatur bis auf 30 °C ansteigen ließ. Die Phasen wurden getrennt und die wässrige Phase wurde zweimal mit Dichlormethan (1500 mL) extrahiert. Die vereinigten organischen Phasen wurden mit Wasser (8000 mL) reextrahiert, mit Na₂SO₄ getrocknet und unter vermindrerten Druck eingedampft. Man erhielt etwa 360 g Rohprodukt als beige Kristalle. Diese wurden in Dichlormethan aufgenommen und über eine Kieselgelschicht (15 x 30 cm, 70-200 µm) filtriert, mit Dichlormethan nachgespült und das Filtrat eingedampft. Man erhielt 270 g (96 %) (4'-Chlorsulfonyl-biphenyl-4-yl)-carbaminsäure isopropylester als gelbe Kristalle.
¹H-NMR (400 MHz, CDCl₃): δ = 1.33 (d, J= 6 Hz, 6 H), 5.05 (heptett, J= 6 Hz, 1 H), 6.68 (bs, 1 H), 7.53 (d, J= 9 Hz, 2 H), 7.59 (d, J= 9 Hz, 2 H), 7.88 (d, J= 9 Hz, 2 H), 8.08 (d, J= 9 Hz, 2 H) ppm.

### Beispiel 4

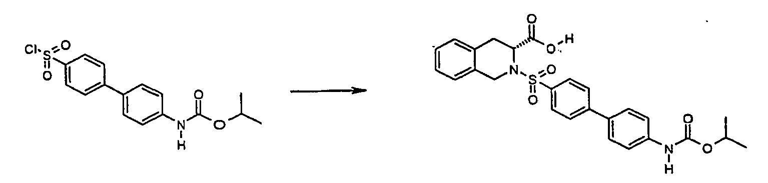

### Herstellung von (R)-2-(4'-Isöpropoxycarbonylamino-biphenyl-4-sulfonyl)-1,2,3,4-tetrahydro-isochinolin-3- carbonsäure

(R)-1,2,3,4-Tetrahydro-isochinolin-3-carbonsäure (139 g, 0,78 Mol) wurden in wasserfreiem Acetonitril (3000 mL) unter trockenem Stickstoff suspendiert und bei 20 °C unter Rühren wurde N,O-Bis-(trimethylsilyl)-acetamid (334 g, 400 mL, 1,64 Mol) innerhalb von 5 Min. zugegeben. Das Gemisch wurde am Rückfluss 1,5 h lang erhitzt und dann auf 74 °C abkühlen gelassen. Anschließend wurde unter Rühren (4'-Chlorsulfonyl-biphenyl-4-yl)-carbaminsäure isopropylester (277 g, 0,78 Mol) innerhalb von 10 Min. in 8 Portionen zugegeben, wobei Gasentwicklung beobachtet wurde. Das gebildete niedrigsiedende Trimethylsilylchlorid wurde abdestilliert und das Gemisch danach weitere 2,5 h am Rückfluss erhitzt. Anschließend wurde auf RT abgekühlt, unter starkem Rühren in ein Gemisch aus Wasser (10 L), Citronensäure (80 g) und Ethylacetat (1500 mL) gegossen und die Phasen getrennt. Die wässrige Phase wurde zweimal mit Ethylacetat (je 1000 mL) extrahiert und die vereinigten organischen Phasen wurden mit Wasser (7 L) reextrahiert. Nach Trocknen über Na₂SO₄ wurde unter verminderten Druck eingedampft. Man erhielt 506 g beiges, kristallines Rohprodukt, das durch Umkristallisieren aus Dichlormethan (1000 mL) gereinigt wurde.
Ausbeute 316 g (78 %) (R)-2-(4'-Isopropoxycarbonylamino-biphenyl-4-sulfonyl)-1,2,3,4-tetrahydro-isochinolin-3- carbonsäure, farblose Kristalle, Fp. 133 °C bis 135 °C (Erweichung),
173 °C bis -175 °C (Schmelzen unter Gasentwicklung).
¹H-NMR (400 MHz, DMSO-d6): δ = 1.27 (d, J= 6 Hz, 6 H), 3.04 (dd, J₁= 16 Hz, J₂= 6 Hz, 1 H), 3.11 (dd, J₁=16 Hz, J₂=3 Hz, 1 H), 4.49 (d, J= 16 Hz, 1 H), 4.61 (d, J= 16 Hz, 1 H), 4.85-4.95 (sh, 2 H), 7.15 (m, 4 H), 7.59 (d, J= 9 Hz, 1 H), 7.67 (d, J= 9 Hz, 1 H), 7.81 (d, J= 9 Hz, 1 H), 7.86 (d, J= 9 Hz, 1 H), 9.74 (s, 1 H), 12.86 (bs, 1 H) ppm. Analyse: C₂₆H₂₆N₂O₆S (494.57): berechnet C 63.14, H 5.30, N 5.66; gefunden C 62.81, H 5.57, N 5.49.

Bestimmung der Enantiomerenreinheit durch HPLC an chiraler Phase: Chiralpak AD-H/39 250x4,6, Laufmittel Heptan/Methanol/Ethanol 8:1:1 + 1% NH₄OAc, Retentionszeit von 8(R)-1,2,3,4-Tetrahydro-isochinolin-3-carbonsäure] = 7,349 Min. (99,97 %),
Retentionszeit von [(S)-1,2,3,4-Tetrahydro-isochinolin-3-carbonsäure] = 8,521 Min. (0,03 %).
Enantiomerenreinheit 99,94 % ee.

## Patentansprüche

1. Verbindung der Formel II, 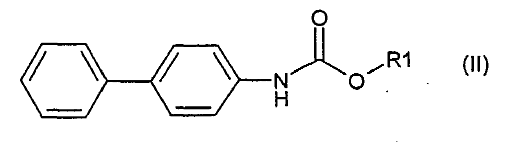 und/oder alle stereoisomeren Formen der Verbindung der Formel II und/oder Gemische diese Formen in jedem Verhältnis, und/oder ein physiologisch verträgliches Salz der Verbindung der Formel II, wobei
R1 für
1. -(C₁-C₁₀)-Alkyl, worin Alkyl gerade oder verzweigt ist,
2. -(C₂-C₁₀)-Alkenyl, worin Alkenyl gerade oder verzweigt ist,
3. -(C₂-C₁₀)-Alkinyl, worin Alkinyl gerade oder verzweigt ist,
4. -(C₁-C₄)-Alkyl-Phenyl,
5. -(C₁-C₄)-Alkyl-(C₃-C₇)-Cycloalkyl,
6. -(C₃-C₇)-Cycloalkyl oder
7. -CH₂CF₃ steht.

2. Verbindung der Formel III, 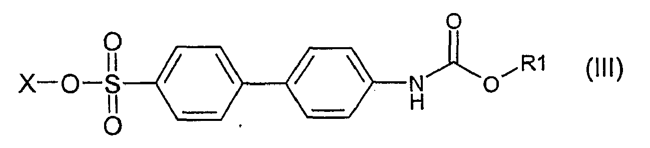 und/oder alle stereoisomeren Formen der Verbindung der Formel III und/oder Gemische diese Formen in jedem Verhältnis, und/oder ein physiologisch verträgliches Salz der Verbindung der Formel III, wobei
R1 für
1. -(C₁-C₁₀)-Alkyl, worin Alkyl gerade oder verzweigt ist,
2. -(C₂-C₁₀)-Alkenyl, worin Alkenyl gerade oder verzweigt ist,
3. -(C₂-C₁₀)-Alkinyl, worin Alkinyl gerade oder verzweigt ist,
4. -(C₁-C₄)-Alkyl-Phenyl,
5. -(C₁-C₄)-Alkyl-(C₃-C₇)-Cycloalkyl,
6. -(C₃-C₇)-Cycloalkyl oder
7. -CH₂CF₃ steht und X für Li, Na, K, Rb, Cs steht.

3. Verbindung der Formel IV, 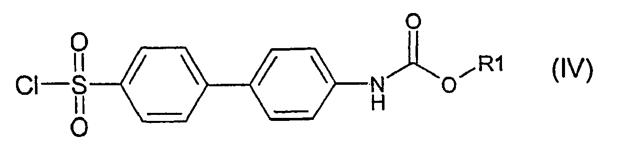 und/oder alle stereoisomeren Formen der Verbindung der Formel IV und/oder Gemische diese Formen in jedem Verhältnis, und/oder ein physiologisch verträgliches Salz der Verbindung der Formel IV, wobei R1 für
1. -(C₁-C₁₀)-Alkyl, worin Alkyl gerade oder verzweigt ist,
2. -(C₂-C₁₀)-Alkenyl, worin Alkenyl gerade oder verzweigt ist,
3. -(C₂-C₁₀)-Alkinyl, worin Alkinyl gerade oder verzweigt ist,
4. -(C₁-C₄)-Alkyl-Phenyl,
5. -(C₁-C₄)-Alkyl-(C₃-C₇)-Cycloalkyl,
6. -(C₃-C₇)-Cycloalkyl oder
7. -CH₂CF₃ steht.

4. Verfahren zur Gewinnung der Verbindung der Formel I 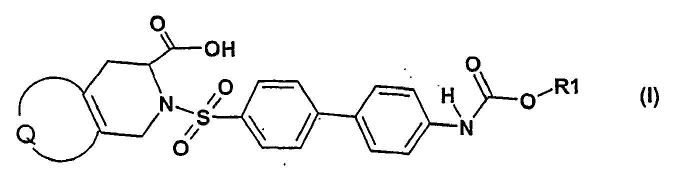 und/oder alle stereoisomeren Formen der Verbindung der Formel I und/oder Gemische diese Formen in jedem Verhältnis, und/oder ein physiologisch verträgliches Salz der Verbindung der Formel I, wobei
R1 für
1. -(C₁-C₁₀)-Alkyl, worin Alkyl gerade oder verzweigt ist,
2. -(C₂-C₁₀)-Alkenyl, worin Alkenyl gerade oder verzweigt ist,
3. -(C₂-C₁₀)-Alkinyl, worin Alkinyl gerade oder verzweigt ist,
4. -(C₁-C₄)-Alkyl-Phenyl,
5. -(C₁-C₄)-Alkyl-(C₃-C₇)-Cycloalkyl,
6. -(C₃-C₇)-Cycloalkyl oder
8. -CH₂CF₃ steht und
und worin 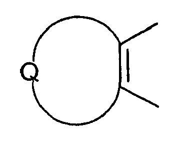 ein Rest aus der Reihe 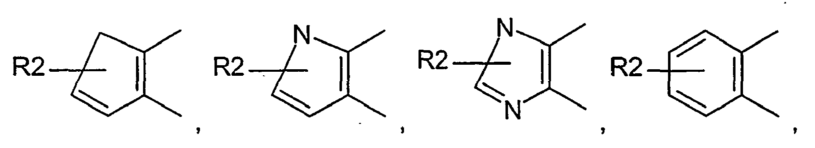 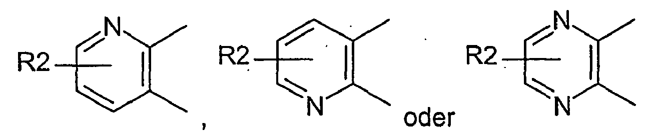 ist und unsubstituiert oder ein-, zwei- oder dreifach substituiert ist durch R2, und R2 für Wasserstoffatom oder -(C₁-C₄)-Alkyl steht, **dadurch gekennzeichnet, dass** man
a) eine Verbindung der Formel II 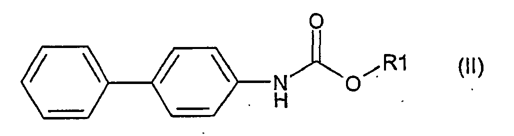 worin R1 die gleiche Bedeutung wie in der Formel I hat,
mit einem Sulfonierungsreagenz in einem organischen Lösungsmittel umsetzt und mit einer wässrigen Alkalimetallsalz-Lösung in eine Verbindung der Formel III überführt, 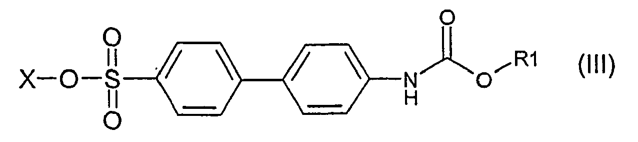 worin X für ein Alkalimetall oder Wasserstoff steht, und
b) die Verbindung der Formel III mit einem Chlorierungsreagenz in einem organischen Lösungsmittel II in Anwesenheit einer organischen Base in eine Verbindung der Formel IV überführt, 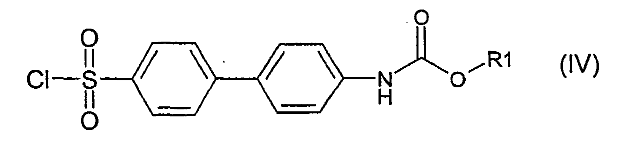 und
c) eine Verbindung der Formel V 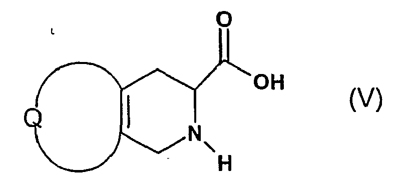 in einem organischen Lösungsmittel III mit einem Silylierungsmittel umsetzt und anschließend mit der Verbindung der Formel IV in eine Verbindung der Formel I überführt.

5. Verfahren gemäß Anspruch 4, **dadurch gekennzeichnet, dass** als Sulfonierungsreagenz Chlorsulfonsäure, Schwefelsäure, Sulfurylchlorid, rauchende Schwefelsäule oder Schwefeltrioxid,
als organisches Lösungsmittel Chloroform, Dichlormethan, Pentan, Heptan, Hexan, Tetrachlormethan, Benzol Toluol, Xylol, Chlorbenzol, 1,2-Dichlorethan oder Trichlorethylen,
als wässrigen Alkalimetallsalz-Lösung Lösungen von wasserlöslichen Alkalimetallsalzen wie NaCl, KCl, LiCl, RbCl oder CsCl oder Alkalimetallsulfate wie Na₂SO₄, K₂SO₄, Li₂SO₄, Rb₂SO₄ oder Cs₂SO₄,
als organischen Lösungsmittel II Chloroform, Dichlormethan, Pentan, Heptan, Hexan, Tetrachlormethan, Toluol, Benzol, Xylol, Chlorbenzol, 1,2-Dichlorethan oder Trichlorethylen,
als organische Base Chinolin, Morpholin, Piperidin, Pyridin, Triethylamin, Picolin oder Lutidin,
als Chlorierungsreagenz Verbindungen zur Umsetzung von Carbonsäuren und Sulfonsäuren zu Carbonsäure- oder Sulfonsäurechloriden wie PCl₅, POCl₃, SOCl₂, Triphenylphosphin in CCl₄ oder Gemische der Chlorierungsreagenzien, als organischen Lösungsmittel III Acetonitril, Dichlormethan, Chloroform, Tetrahydrofuran, Toluol, Dimethoxyethan, Dioxan oder Diethylenglycoldimethylether,
als Silylierungsmittel Verbindungen wie N,O-Bis(trimethylsilyl)-acetamid oder Trimethylsilylchlorid oder Gemische der Silylierungsmittel
eingesetzt werden.

6. Verfahren gemäß Anspruch 4 oder 5, **dadurch gekennzeichnet, dass** man auf 1 Mol der Verbindung der Formel II von 1 Mol bis 10 Mol, insbesondere von 1 Mol bis 3 Mol, des Sulfonierungsreagenz einsetzt.

7. Verfahren gemäß einem oder mehreren der Ansprüche 4 bis 6, **dadurch gekennzeichnet, dass** man auf 1 Mol der Verbindung der Formel III von 0,1 Mol bis 5 Mol, insbesondere von 0,1 Mol bis 1 Mol, der organischen Base einsetzt.

8. Verfahren gemäß einem oder mehreren der Ansprüche 4 bis 7, **dadurch gekennzeichnet, dass** man auf 1 Mol der Verbindung der Formel III von 1 Mol bis 5 Mol, insbesondere von 1 Mol bis 2 Mol, des Chlorierungsreagenzes einsetzt.

9. Verfahren gemäß einem oder mehreren der Ansprüche 4 bis 8, **dadurch gekennzeichnet, dass** man auf 1 Mol der Verbindung der Formel IV von 0,5 Mol bis 2 Mol, insbesondere von 0,9 Mol bis 1,1 Mol, der Verbindung der Formel V einsetzt.

10. Verfahren gemäß einem oder mehreren der Ansprüche 4 bis 8, **dadurch gekennzeichnet, dass** man auf 1 Mol der Verbindung der Formel V von 1 Mol bis 5 Mol, insbesondere von 2 Mol bis 2,5 Mol, des Silylierungsmittels einsetzt.

## Claims

1. A compound of the formula II 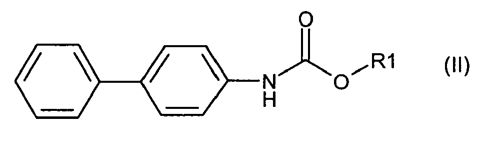 and/or all stereoisomeric forms of the compound of the formula II and/or mixtures of these forms in any ratio, and/or a physiologically tolerated salt of the compound of the formula II, where
R1 is
1. -(C₁-C₁₀)-alkyl where alkyl is linear or branched,
2. -(C2-C10)-alkenyl where alkenyl is linear or branched,
3. -(C₂-C₁₀)-alkynyl where alkynyl is linear or branched,
4. -(C₁-C₄)-alkylphenyl,
5. -(C₁-C₄)-alkyl-(C₃-C₇)-cycloalkyl,
6. -(C₃-C₇)-cycloalkyl or
7. -CH₂CF₃.

2. A compound of the formula III 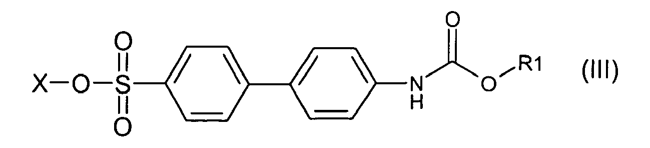 and/or all stereoisomeric forms of the compound of the formula III and/or mixtures of these forms in any ratio, and/or a physiologically tolerated salt of the compound of the formula III, where
R1 is
1. -(C₁-C₁₀)-alkyl where alkyl is linear or branched,
2. -(C₂-C₁₀)-alkenyl where alkenyl is linear or branched,
3. -(C₂-C₁₀)-alkynyl where alkynyl is linear or branched,
4. -(C₁-C₄)-alkylphenyl,
5. -(C₁-C₄)-alkyl-(C₃-C₇)-cycloalkyl,
6. -(C₃-C₇)-cycloalkyl or
7. -(CH₂CF₃ and
X is Li, Na, K, Rb, Cs.

3. A compound of the formula IV 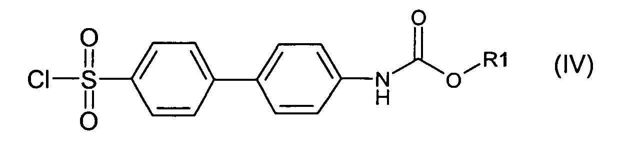 and/or all stereoisomeric forms of the compound of the formula IV and/or mixtures of these forms in any ratio, and/or a physiologically tolerated salt of the compound of the formula IV, where
R1 is
1. -(C₁-C₁₀)-alkyl where alkyl is linear or branched,
2. -(C₂-C₁₀)-alkenyl where alkenyl is linear or branched,
3. -(C₂-C₁₀)-alkynyl where alkynyl is linear or branched,
4. -(C₁-C₄)-alkylphenyl,
5. -(C₁-C₄)-alkyl-(C₃-C₇)-cycloalkyl,
6. -(C₃-C₇)-cycloalkyl or
7. -CH₂CF₃.

4. A process for obtaining the compound of the formula I 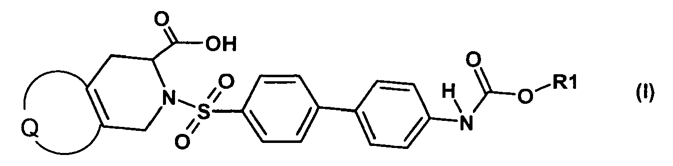 and/or all stereoisomeric forms of the compound of the formula I and/or mixtures of these forms in any ratio, and/or a physiologically tolerated salt of the compound of the formula I, where
R1 is
1. -(C₁-C₁₀)-alkyl where alkyl is linear or branched,
2. -(C₂-C₁₀)-alkenyl where alkenyl is linear or branched,
3. -(C₂-C₁₀)-alkynyl where alkynyl is linear or branched,
4. -(C₁-C₄)-alkylphenyl,
5. -(C₁-C₄)-alkyl-(C₃-C₇)-cycloalkyl,
6. -(C₃-C₇)-cycloalkyl or
7. -CH₂CF₃,
and where 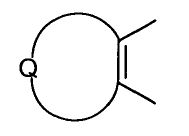 is a radical from the group of 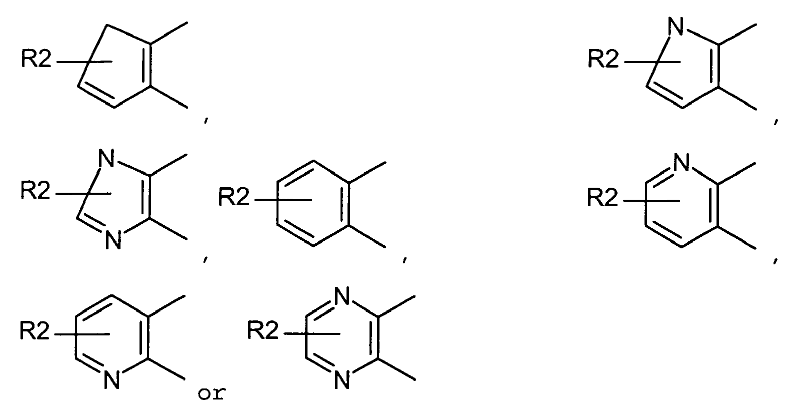 and is unsubstituted or mono-, di- or trisubstituted by R2, and
R2 is a hydrogen atom or -(C₁-C₄)-alkyl, which comprises
a) reacting a compound of the formula II 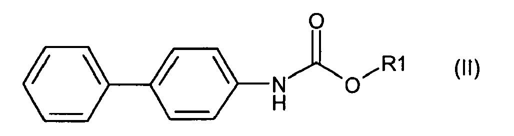 where R1 is as defined in the formula I with a sulfonating reagent in an organic solvent and converting it using an aqueous alkali metal salt solution to a compound of the formula III 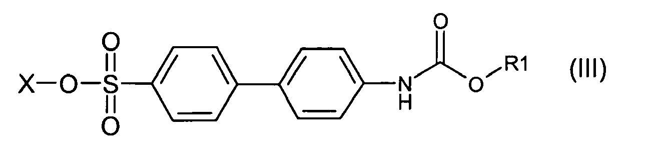 where X is an alkali metal or hydrogen, and
b) converting the compound of the formula III with a chlorinating reagent in an organic solvent II in the presence of an organic base to a compound of the formula IV 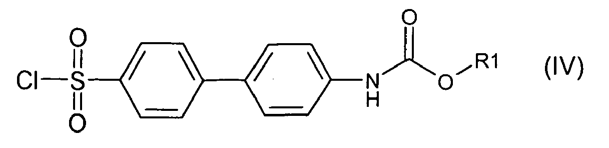 and
c) reacting a compound of the formula V 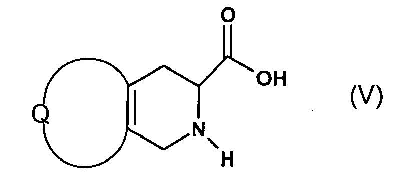 in an organic solvent III with a silylating agent and subsequently converting it using the compound of the formula IV to a compound of the formula I.

5. The process as claimed in claim 4, wherein the sulfonating reagent used is chlorosulfonic acid, sulfuric acid, sulfuryl chloride, fuming sulfuric column or sulfur trioxide,
the organic solvent used is chloroform, dichloromethane, pentane, heptane, hexane, tetrachloromethane, benzene, toluene, xylene, chlorobenzene, 1,2-dichloroethane or trichloroethylene,
the aqueous alkali metal salt solution used is a solution of water-soluble alkali metal salts such as NaCl, KCl, LiCl, RbCl or CsCl or alkali metal sulfates such as Na₂SO₄, K₂SO₄, Li₂SO₄, Rb₂SO₄ or Cs₂SO₄,
the organic solvent II used is chloroform, dichloromethane, pentane, heptane, hexane, tetrachloromethane, toluene, benzene, xylene,
chlorobenzene, 1,2-dichloroethane or trichloroethylene,
the organic base used is quinoline, morpholine, piperidine, pyridine, triethylamine, picoline or lutidine,
the chlorinating reagent used is a compound to convert carboxylic acids and sulfonic acids to carbonyl or sulfonyl chlorides such as PCl₅, POCl₃, SOCl₂, triphenylphosphine in CCl₄ or mixtures of the chlorinating reagents,
the organic solvent III used is acetonitrile, dichloromethane, chloroform, tetrahydrofuran, toluene, dimethoxyethane, dioxane or diethylene glycol dimethyl ether,
the silylating agent used is a compound such as N,O-bis(trimethylsilyl)acetamide or trimethylsilyl chloride or a mixture of the silylating agents.

6. The process as claimed in claim 4 or 5, wherein from 1 mol to 10 mol, in particular from 1 mol to 3 mol, of the sulfonating reagent are used for 1 mol of the compound of the formula II.

7. The process as claimed in one or more of claims 4 to 6, wherein from 0.1 mol to 5 mol, in particular from 0.1 mol to 1 mol, of the organic base are used for 1 mol of the compound of the formula III.

8. The process as claimed in one or more of claims 4 to 7, wherein from 1 mol to 5 mol, in particular from 1 mol to 2 mol, of the chlorinating reagent are used for 1 mol of the compound of the formula III.

9. The process as claimed in one or more of claims 4 to 8, wherein from 0.5 mol to 2 mol, in particular from 0.9 mol to 1.1 mol, of the compound of the formula V are used for 1 mol of the compound of the formula IV.

10. The process as claimed in one or more of claims 4 to 8, wherein from 1 mol to 5 mol, in particular from 2 mol to 2.5 mol, of the silylating agent are used for 1 mol of the compound of the formula V.

## Revendications

1. Composé de formule II, 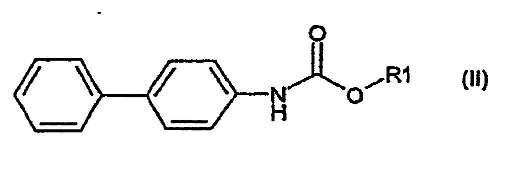 et/ou toutes les formes stéréoisomères du composé de formule II et/ou les mélanges de ces formes selon tout rapport, et/ou sel physiologiquement compatible du composé de formule II, dans lequel
R1 représente
1. un radical -alkyle en C₁ à C₁₀, le radical alkyle étant à chaîne droite ou ramifiée,
2. un radical -alcényle en C₂ à C₁₀, le radical alcényle étant à chaîne droite ou ramifiée,
3. un radical -alcynyle en C₂ à C₁₀, le radical alcynyle étant à chaîne droite ou ramifiée,
4. un radical -(alkyle en C₁ à C₄)-phényle,
5. un radical -(alkyle en C₁ à C₄)-(cycloalkyle en C₃ à C₇),
6. un radical -cycloalkyle en C₃ à C₇, ou
7. un radical -CH₂CF₃.

2. Composé de formule III, 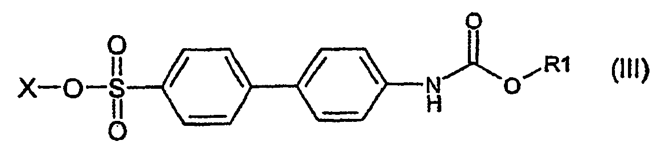 et/ou toutes les formes stéréoisomères du composé de formule III et/ou les mélanges de ces formes selon tout rapport, et/ou sel physiologiquement compatible du composé de formule III, dans lequel
R1 représente
1. un radical -alkyle en C₁ à C₁₀, le radical alkyle étant à chaîne droite ou ramifiée,
2. un radical -alcényle en C₂ à C₁₀, le radical alcényle étant à chaîne droite ou ramifiée,
3. un radical -alcynyle en C₂ à C₁₀, le radical alcynyle étant à chaîne droite ou ramifiée,
4. un radical -(alkyle en C₁ à C₄)-phényle,
5. un radical -(alkyle en C₁ à C₄)-(cycloalkyle en C₃ à C₇),
6. un radical -cycloalkyle en C₃ à C₇, ou
7. un radical -CH₂CF₃, et
X représente Li, Na, K, Rb, Cs.

3. Composé de formule IV, 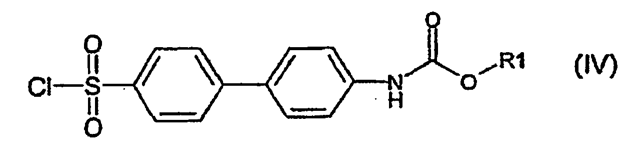 et/ou toutes les formes stéréoisomères du composé de formule IV et/ou les mélanges de ces formes selon tout rapport, et/ou sel physiologiquement compatible du composé de formule IV, dans lequel
R1 représente
1. un radical -alkyle en C₁ à C₁₀, le radical alkyle étant à chaîne droite ou ramifiée,
2. un radical -alcényle en C₂ à C₁₀, le radical alcényle étant à chaîne droite ou ramifiée,
3. un radical -alcynyle en C₂ à C₁₀, le radical alcynyle étant à chaîne droite ou ramifiée,
4. un radical -(alkyle en C₁ à C₄)-phényle,
5. un radical - (alkyle en C₁ à C₄) - (cycloalkyle en C₃ à C₇),
6. un radical -cycloalkyle en C₃ à C₇, ou
7. un radical -CH₂CF₃.

4. Procédé de préparation du composé de formule I 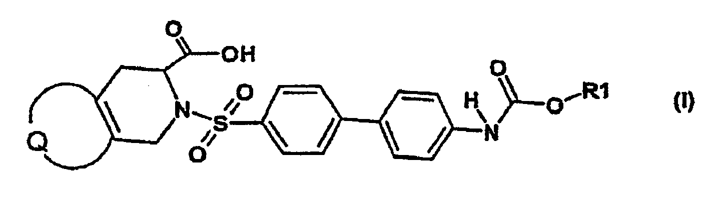 et/ou de toutes les formes stéréoisomères du composé de formule I et/ou mélanges de ces formes selon tout rapport et/ou sel physiologiquement compatible du composé de formule I, dans lequel
R1 représente
1. un radical -alkyle en C₁ à C₁₀, le radical alkyle étant à chaîne droite ou ramifiée,
2. un radical -alcényle en C₂ à C₁₀, le radical alcényle étant à chaîne droite ou ramifiée,
3. un radical -alcynyle en C₂ à C₁₀, le radical alcynyle étant à chaîne droite ou ramifiée,
4. un radical -(alkyle en C₁ à C₄)-phényle,
5. un radical -(alkyle en C₁ à C₄)-(cycloalkyle en C₃ à C₇),
6. un radical -cycloalkyle en C₃ à C₇, ou
7. un radical -CH₂CF₃ et
et dans lequel 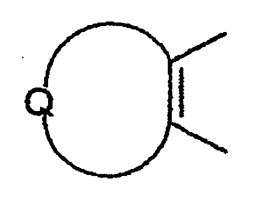 est un radical de la série 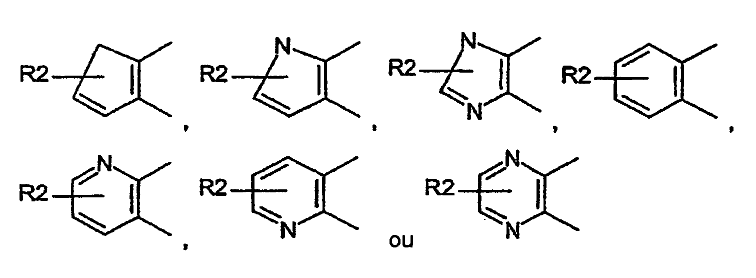 et est non substitué, ou est une fois, deux fois ou trois fois substitué par R2, et
R2 est un atome d'hydrogène ou un radical -alkyle en C₁ à C₄, **caractérisé en ce que**
a) on fait réagir un composé de formule II 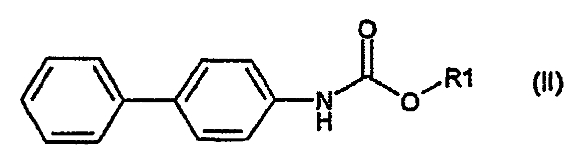 dans laquelle R1 a les mêmes significations que dans la formule I,
avec un réactif de sulfonation dans un solvant organique, et on le convertit, avec une solution aqueuse d'un sel d'un métal alcalin, en un composé de formule III 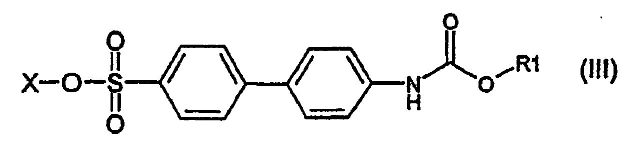 dans laquelle X est un métal alcalin ou un atome d'hydrogène, et
b) on convertit le composé de formule III avec un réactif de chloration dans un solvant organique II, en présence d'une base organique, en un composé de formule IV 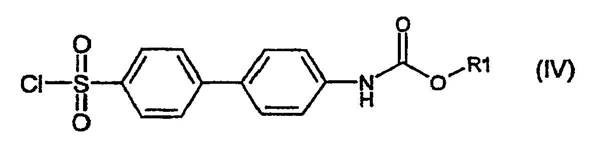 et
c) on fait réagir un composé de formule V 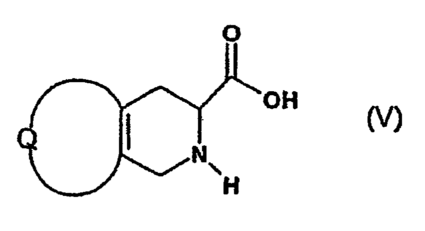 dans un solvant organique III avec un agent de silylation, puis on le convertit avec le composé de formule IV en un composé de formule I.

5. Procédé selon la revendication 4, **caractérisé en ce qu'**on utilise :
en tant que réactif de sulfonation, l'acide chlorosulfonique, l'acide sulfurique, le chlorure de sulfuryle, l'acide sulfurique fumant ou le trioxyde de soufre,
en tant que solvant organique, le chloroforme, le dichlorométhane, le pentane, l'heptane, l'hexane, le tétrachlorométhane, le benzène, le toluène, le xylène, le chlorobenzène, le 1,2-dichloroéthane ou le trichloréthylène,
en tant que solution aqueuse d'un sel d'un métal alcalin, des solutions de sels solubles dans l'eau d'un métal alcalin tels que NaCl, KCl, LiCl, RbCl ou CsCl, ou des sulfates de métaux alcalins tels que Na₂SO₄, K₂SO₄, Li₂SO₄, Rb₂SO₄ ou Cs₂SO₄,
en tant que solvant organique II, le chloroforme, le dichlorométhane, le pentane, l'heptane, l'hexane, le tétrachlorométhane, le toluène, le benzène, le xylène, le chlorobenzène, le 1,2-dichloroéthane ou le trichloréthylène,
en tant que base organique, la quinoléine, la morpholine, la pipéridine, la pyridine, la triéthylamine, la picoline ou la lutidine,
en tant que réactif de chloration, les composés destinés à convertir des acides carboxyliques et des acides sulfoniques en chlorures de carbonyle ou de sulfonyle tels que PCl₅, POCl₃, SOCl₂, la triphénylphosphine dans du CCl₄, ou les mélanges de réactifs de chloration,
en tant que solvant organique III, l'acétonitrile, le dichlorométhane, le chloroforme, le tétrahydrofuranne, le toluène, le diméthoxyéthane, le dioxanne ou l'éther diméthylique du diéthylèneglycol,
en tant qu'agent de silylation, les composés tels que le N,O-bis(triméthylsilyl)-acétamide ou le chlorure de triméthylsilyle ou les mélanges des agents de silylation.

6. Procédé selon la revendication 4 ou 5, **caractérisé en ce qu'**on utilise par mole du composé de formule II de 1 à 10 moles, en particulier de 1 à 3 moles du réactif de sulfonation.

7. Procédé selon l'une ou plusieurs des revendications 4 à 6, **caractérisé en ce qu'**on utilise par mole du composé de formule III de 0,1 à 5 moles, en particulier de 0,1 mole à 1 mole de la base organique.

8. Procédé selon l'une ou plusieurs des revendications 4 à 7, **caractérisé en ce qu'**on utilise par mole du composé de formule III de 1 mole à 5 moles, en particulier de 1 mole à 2 moles du réactif de chloration.

9. Procédé selon l'une ou plusieurs des revendications 4 à 8, **caractérisé en ce qu'**on utilise par mole du composé de formule IV de 0,5 mole à 2 moles, en particulier de 0,9 mole à 1,1 moles du composé de formule V.

10. Procédé selon l'une ou plusieurs des revendications 4 à 8, **caractérisé en ce qu'**on utilise par mole du composé de formule V de 1 mole à 5 moles, en particulier de 2 moles à 2,5 moles de l'agent de silylation.
